Europäisches Patentamt

European Patent Office

Office européen des brevets

(11) Veröffentlichungsnummer: **0 471 309 A1**

## EUROPÄISCHE PATENTANMELDUNG

(21) Anmeldenummer: **91113420.3**

(22) Anmeldetag: **09.08.91**

(51) Int. Cl.5: **A61K 31/12**, A61K 9/107, A61K 47/24, A61K 47/28

(30) Priorität: **17.08.90 CH 2678/90**

(43) Veröffentlichungstag der Anmeldung:
**19.02.92 Patentblatt 92/08**

(84) Benannte Vertragsstaaten:
**AT BE CH DE DK FR GB IT LI LU NL SE**

(71) Anmelder: **F. HOFFMANN-LA ROCHE AG**
**Postfach 3255**
**CH-4002 Basel(CH)**

(72) Erfinder: **Amédée-Manesme, Oliver**
**11 Parc Alexandre Dumas**
**F-78160 Marly-le-Roi(FR)**
Erfinder: **Grüter, Josef**
**Brunngasse 12**
**CH-4202 Duggingen(CH)**
Erfinder: **Hanck, Alfred**
**Kilchgrundstrasse 10**
**CH-4125 Riehen(CH)**

(74) Vertreter: **Lederer, Franz, Dr. et al**
**Lederer, Keller & Riederer, Patentanwälte,**
**Lucile-Grahn-Strasse 22**
**W-8000 München 80(DE)**

(54) **Verwendung von Mischmicellen.**

(57) Verwendung von Mischmicellen zur Herstellung peroraler Applikationsformen von Vitamin K1.

EP 0 471 309 A1

Vitamin-K-Mangelblutungen bei Neugeborenen (3.-5. Lebenstag) wurden in den letzten Jahren, dank neuer diagnostischer Möglichkeiten, immer häufiger diagnostiziert. Gleichzeitig wurden seit 1980 immer häufiger bei bis anhin gesund erscheinenden Säuglingen späte Vitamin-K-Mangelblutungen (in der 3.-8. Lebenswoche) beobachtet.

Eine effektive Behandlung dieser Krankheit war, mangels geeigneter Applikationsformen, nur mit grossem Risiko für das Neugeborene möglich.

Bislang wurde Vitamin K1 zur Verabreichung an Neugeborene mit Hilfe synthetischer Lösungsvermittler wie z.B. Cremophor EL, Tween und Pluronics in Lösung gebracht. Diese synthetischen Micellbildner können bei der parenteralen Applikation zuweilen verschiedene Nebenwirkungen, wie z.B. allergische Reaktionen, anaphylaktische Schocks, oder Hämolyse verursachen.

Im Rahmen der vorliegenden Erfindung wurde überraschend gefunden, dass eine sichere und effektive Behandlung mit Vitamin K1 auf oralem Weg in Form wässriger Mischmicell-Lösungen erreicht werden kann.

Die Erfindung betrifft somit die Verwendung von Mischmicellen zur Herstellung peroraler Applikationsformen für Vitamin K1, insbesondere zur Anwendung in der Neonatologie.

Als Mischmicellen können wässrige Lösungen von Gallensäuresalzen und Phospholipiden verwendet werden, wie sie beispielsweise aus der DE-OS 2 730 570 bekannt sind.

Als Gallensäuren kommen für die erfindungsgemässen Lösungen Trihydroxycholansäuren, wie Cholsäure, Glycocholsäure, Taurocholsaure, und Dihydroxycholansäuren, wie Deoxycholsäure, Glycodeoxycholsäure, Taurodeoxycholsäure, Chenodeoxysäure, Glycochenodeoxycholsäure und Taurochenodeoxycholsäure, in Betracht. Bevorzugt ist Glycocholsäure. Als Gallensäuresalze kommen insbesondere Alkalisalze, wie das Natriumsalz, in Betracht.

Beispiele von Phospholipiden sind Phosphatide, wie Phosphatidylcholine, Glycerinäther-Phosphatide, Phosphatidyläthanolamin, Phosphatidylinosit, Phosphatidylserin, Plasmologene oder Sphingomyeline. Bevorzugt sind Phosphtatidylcholine, wie Soja- und Eilecithin.

Das Molverhältnis von Lipid zu Gallensäuresalz liegt zweckmässig zwischen 0,8:1 und 1,5:1.

Der Anteil von Lipid plus Gallensäuresalz in der Lösung kann über weite Grenzen variieren und z.B. 50-150 mg/ml betragen.

Der Anteil des Vitamin K1 in den Mischmicell-Lösungen kann ebenfalls über weite Grenzen variieren und z.B. 1-100 mg/ml Lösung betragen.

Die Mischmicell-Lösungen können durch blosses Auflösen und Vermischen der einzelnen Bestandteile hergestellt werden.

In einer speziellen Ausführungsform kann man zunächst eine wässrige Mischmicell-Lösung aus einem Lipid und einem Gallensäuresalz herstellen und sodann das Vitamin K1 zugeben.

Beispielsweise wird Glycocholsäure in gereinigtem Wasser Ph.Eur. mit Hilfe von Natriumhydroxid gelöst und das pH mit Salzsäure auf einen Wert von 5,5 eingestellt. Das Lecithin wird in der Natriumglycocholat-Lösung unter Rühren gelöst und das Vitamin K1 unter Rühren beigefügt.

In die so erhaltene Vitamin K1-Mischmicellenlösung wird gereinigtes Wasser Ph.Eur. gegeben, das pH mit Salzsäure auf einen Wert von 6,0 eingestellt und mit gereinigtem Wasser Ph.Eur. auf das Endvolumen ergänzt.

Die erhaltene Lösung wird zweckmässig durch ein geeignetes Filter filtriert und in geeignete Behältnisse abgefüllt.

Das nachstehende Beispiel erläutert die Herstellung oraler Vitamin K1-Präparate gemäss der Erfindung weiter.

Beispiel

Zusammensetzung eines Vitamin K1-Präparats zur oralen Verabreichung:

| Bestandteil | pro ml |
|---|---|
| Vitamin K1 | 10,0 mg |
| Glycocholsäure | 54,6 mg |
| Lecithin | 75,6 mg |
| NaOH reinst | q.s. |

```
Salzsäure zur Analyse                    q.s.

Wasser für Injektionszwecke          ad  1,0 ml
```

Herstellung:

a) 1,5 kg Natriumhydroxid werden in 70,0 kg gereinigtem Wasser Ph.Eur. unter $N_2$-Begasung und Kühlung gelöst.

b) In dieser Lösung a) werden 21,84 kg Glycocholsäure suspendiert und durch Zugabe von Natriumhydroxid (10%) bei pH 5,5-6,5 in Lösung gebracht. Nach vollständigem Auflösen der Glycocholsäure wird das pH mit IN Salzsäure auf einen pH-Wert von 5,5 eingestellt.

c) 30,240 kg zerkleinertes Lecithin werden in die auf 60°C erwärmte Glycocholatlösung b) und unter starkem Rühren gelöst.

d) 4,0 kg Vitamin K1 werden langsam in die Mischmicellenlösung c) gegossen und es wird gerührt, bis eine fast klare Lösung entstanden ist.

e) Die Vitamin K1-Mischmicellenlösung d) wird unter Rühren mit 265 kg gereinigtem Wasser Ph.Eur. versetzt und auf 25°C abgekühlt.

f) Die erhaltene Lösung e) wird mit IN Salzsäure auf pH 6,0 ± 0,1 eingestellt und mit gereinigtem Wasser Ph.Eur. auf das Endvolumen von 400 l ergänzt.

g) Die Lösung f) durch eine geeignete Filterkerze (z.B. 0,2-1,2 µm) filtriert, und unter $N_2$-Atmosphäre abgefüllt.

Die unerwartet gute Wirksamkeit der erfindungsgemäss erhältlichen Präparate zeigt sich im Vergleich mit konventiellen Vitamin-K1-Präparaten.

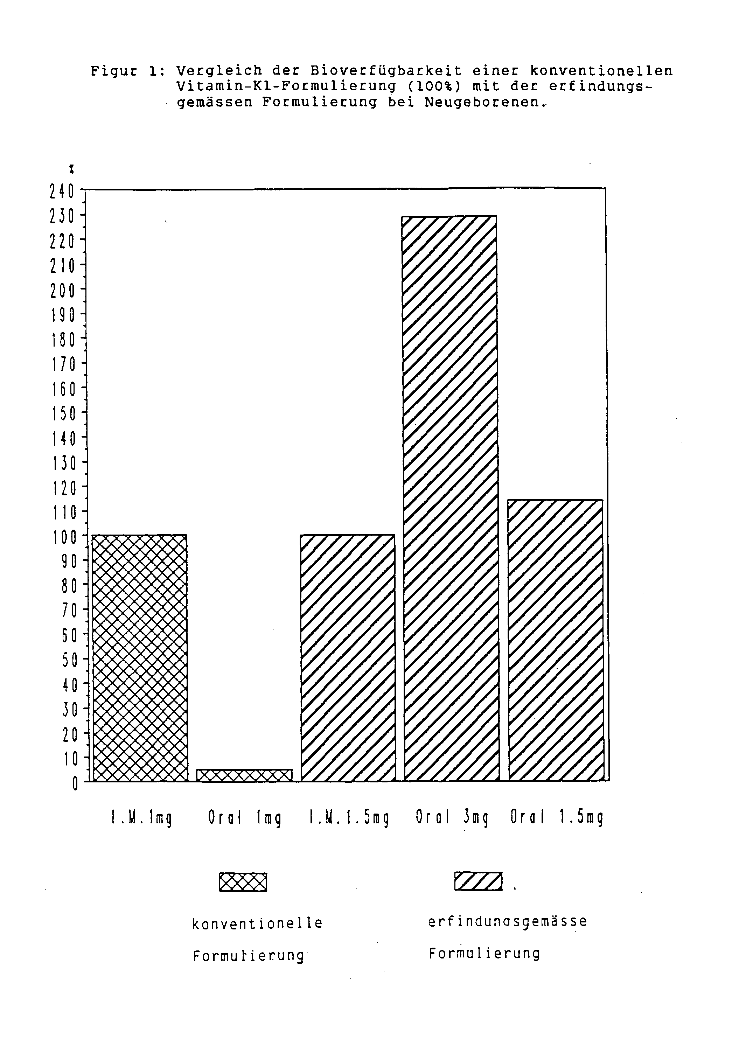

Figur 1 zeigt die Bioverfügbarkeit einer konventionellen Lösung (hergestellt mit "Cremophor EL") und der erfindungsgemässen Mischmicell-Lösung. Als Referenz (100%) dient die Bioverfügbarkeit, die durch intramuskuläre Applikation entsprechender Lösungen erreicht wird.

Bei oraler Applikation einer Lösung mit einem synthetischen Lösungsvermittler (Cremophor EL) betrug die Bioverfügbarkeit von Vitamin K1 lediglich 5% der vergleichbaren i.m. verabreichten Applikationsform (100%).

Mit einer Mischmicell-Lösung wurde dagegen eine orale Bioverfügbarkeit von Vitamin K1 von 115% derjenigen der vergleichbaren i.m. verabreichten Applikationsform erreicht.

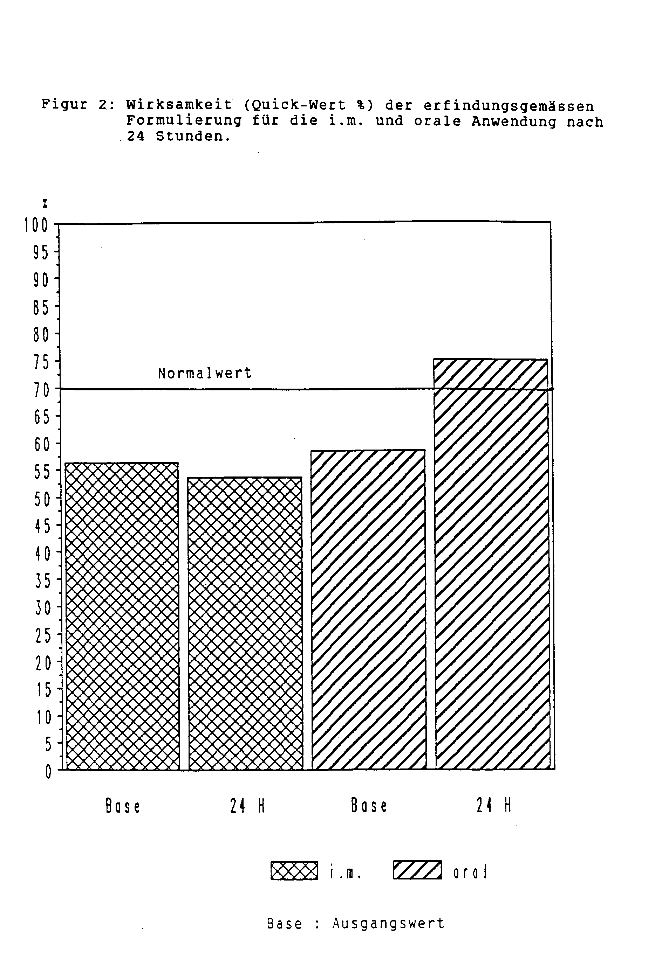

Figur 2 zeigt die im Neugeborenenblut erhaltenen Quick-Werte 24 Stunden nach i.m. Injektion einer Mischmicell-Lösung von Vitamin K1 und nach oraler Verabreichung der K1-Mischmicell-Lösung gemäss der vorliegenden Erfindung, jeweils im Vergleich zum Ausgangswert.

Die i.m.-Form von Vitamin K1 vermochte den Ausgangs-Quick-Wert innert 24 Stunden nach Anwendung (wichtigste Phase der Wirksamkeit) nicht zu steigern, hingegen konnte mit der oralen Anwendungsform der Quick-Wert innert 24 Stunden von ca. 55% auf 75%, also in den Normalbereich angehoben werden (min. Normalbereich = 70%).

**Patentansprüche**

1.  Verwendung von Mischmicellen zur Herstellung peroraler Applikationsformen von Vitamin K1.

2.  Verwendung gemäss Anspruch 1, wobei Mischmicellen aus Gallensäuresalzen und Phospholipiden verwendet werden.

3.  Verwendung gemäss Anspruch 2, wobei das Gallensäuresalz ein Glycocholat und das Phospholipid Lecithin ist.

4.  Verwendung gemäss den Ansprüchen 1-3 zur Anwendung in der Neonatologie.

5.  Handelspackung, enthaltend eine Mischmicell-Lösung von Vitamin K1 zusammen mit Instruktionen zur oralen Verabreichung an Neugeborene.

Figur 1: Vergleich der Bioverfügbarkeit einer konventionellen
Vitamin-K1-Formulierung (100%) mit der erfindungsgemässen Formulierung bei Neugeborenen.

konventionelle
Formulierung

erfindungsgemässe
Formulierung

Figur 2: Wirksamkeit (Quick-Wert %) der erfindungsgemässen
Formulierung für die i.m. und orale Anwendung nach
24 Stunden.

Base : Ausgangswert

**Europäisches Patentamt**

# EUROPÄISCHER RECHERCHENBERICHT

Nummer der Anmeldung

## EP 91 11 3420

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | DE-A-2 730 570 (HOFFMANN-LA ROCHE) <br> * Ansprüche 27-30,32-34,39-40 * <br> – – – | 1-3 | A 61 K 31/12 <br> A 61 K 9/107 <br> A 61 K 47/24 |
| A | WO-A-9 008 534 (SCHERING) <br> * Ansprüche 1-4,6; Seite 3, Zeilen 1-4,15-19; Seite 5, Zeilen 21-22 * <br> – – – | 1-3 | A 61 K 47/28 |
| A | STN INFORMATION SERVICES DATENBANK: CHEMICAL ABSTRACTS, Band 108, Nr. 20, Zusammenfassung Nr. 173492p, Columbus, Ohio, US; G.J. SEWELL et al.: "The formulation and stability of a unit-dose oral vitamin K1 preparation", <br> & J. CLIN. PHARM. THER., 13(1), 73-6 <br> * Zusammenfassung * <br> – – – – – | 4-5 | |

### RECHERCHIERTE SACHGEBIETE (Int. Cl.5)

A 61 K

Der vorliegende Recherchenbericht wurde für alle Patentansprüche erstellt

| Recherchenort | Abschlußdatum der Recherche | Prüfer |
|---|---|---|
| Den Haag | 21 November 91 | SCARPONI U. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

-----------------------------------------------------

& : Mitglied der gleichen Patentfamilie, übereinstimmendes Dokument